# EUROPÄISCHE PATENTSCHRIFT

(11) **EP 1 887 960 B1**
(45) Veröffentlichungstag und Bekanntmachung des Hinweises auf die Patenterteilung: **16.11.2011**
(21) Anmeldenummer: 06753806.6
(22) Anmeldetag: 23.05.2006
(51) Int. Cl.: A61B 19/00

(54) **Nadelpositioniersystem**
Needle positioning system
Système de positionnement d'aiguille

(30) Priorität: 23.05.2005 DE 102005024157
(43) Veröffentlichungstag der Anmeldung: 20.02.2008
(73) Patentinhaber: amedo smart tracking solutions GmbH, 44799 Bochum (DE)
(72) Erfinder: BUSCH, Martin, 58455 Witten (DE); DELI, Martin, 45472 Mühlheim a. d. Ruhr (DE); SPEDER, Jürgen, 44807 Bochum (DE); GROENEMEYER, Dietrich, 45549 Sprockhoevel (DE)
(74) Vertreter: Isfort, Olaf
(86) Internationale Anmeldenummer: PCT/EP2006/004884
(87) Internationale Veröffentlichungsnummer: WO 2006/125605

(56) Entgegenhaltungen:
- US-A- 5 116 344
- US-A- 5 792 215
- US-A- 6 021 342
- US-A- 6 044 291
- US-A- 6 096 049
- US-A1- 2003 167 061
- US-B1- 6 423 076

## Beschreibung

Die Erfindung betrifft eine Vorrichtung zum Positionieren von Instrumenten innerhalb eines Untersuchungsraumes, bei dem gerichtete elektromagnetische Strahlung einen Zugangsbereich und die relative Ausrichtung des Instrumentes zur Erreichung des Zielbereiches, der in der Trajektorie liegt, markiert.

Die Erfindung betrifft ferner ein Verfahren zum Positionieren eines interventionellen Instrumentes innerhalb eines Untersuchungsraumes sowie Instrumente zur Verwendung in dem Verfahren und ein Computerprogramm zur Steuerung einer Positioniervorrichtung.

In der interventionellen Radiologie nimmt die Bedeutung der Mikrotherapie unter Führung von Schnittbildverfahren, wie CT, MR, US als Ersatz für operative Eingriffe eine immer größere Rolle ein. Zwei wichtige Gründe sind zum Einen eine deutliche Reduktion der Kosten im Vergleich zu einem klassischen Eingriff und zum Anderen ein, für den Patienten schonenderer Eingriff. Ein zur Führung des mikrotherapeutischen Eingriffs ideales Bild gebendes Verfahren ist die Kernspintomographie, von der keine Nebenwirkungen bekannt sind. Sie bietet eine freie Schichtpositionierung und ermöglicht als Multi-Parameter-Methode, eine an die spezielle Fragstellung des mikrotherapeutischen Eingriffs angepasste Kontrastauswahl an.

Allerdings ist die anfängliche Euphorie über die möglichen Einsatzgebiete der Kernspintomographie in den letzten Jahren etwas abgeklungen. Verantwortlich sind dafür die erhöhten Kosten der Kernspintomographie im Vergleich zu anderen Schnittbild gebenden Verfahren und der erhöhte Zeitaufwand bei den Eingriffen.

Üblicherweise erfolgt ein mikrotherapeutischer Eingriff heutzutage mit Hilfe der tomographischen Verfahren nach dem nachfolgend genannten Schema. Ein typischer mikrotherapeutische Einsatz umfasst dabei die Medikamentengabe und die mechanische oder thermische Ablation durch eine Nadel oder Kanüle. Dabei wird zunächst der zu therapierende Bereich, d. h. der Zielpunkt mit Hilfe der Tomographie ermittelt. Sodann wird eine Schicht ausgewählt, innerhalb derer der Zugang zum Zielpunkt möglich ist, die dann den Zugangsweg festlegt.

Für einen CT-gestützten mikrotherapeutischen Eingriff bedeutet dies, dass der Winkel der Gantry festgelegt ist, der im Allgemeinen 0°C beträgt. Obwohl im Kernspintomographen eine adäquate Angulierung der Schichten erfolgen kann, ist hier bisher nur eine rein transversale Ausrichtung möglich. Als nächstes wird innerhalb des Datensatzes der ausgewählten Schicht bzw. Schichten ein Zugangsweg zum Zielpunkt der Therapie, ausgehend von der Hautoberfläche, eingezeichnet. Dabei wird der Zugangspunkt, an dem der Einstich auf der Hautoberfläche erfolgt, die Länge des Zugangsweges vom Zugangspunkt bis zum Zielpunkt, sowie der Einstichwinkel den Bildinformationen entnommen.

Im Weiteren wird gestützt auf die Bildinformation der Abstand des Zugangspunktes zu weiteren Referenzpunkten auf der Hautoberfläche, die beispielsweise durch den Dornfortsatz der Wirbelsäule gebildet werden, bestimmt. Im Anschluß wird der Patient auf der Liege des Patiententisches in eine Position gefahren, das ein Positionierlaser die ausgewählte Schicht auf der Körperoberfläche anzeigt. Ausgehend von den anatomischen Referenzpunkten, die durch einen Dornfortsatz der Wirbelsäule gebildet werden, wird der Abstand zum Zugangspunkt in der ausgewählten Schicht mit dem Lineal bestimmt und auf der Haut markiert. Dann erfolgt eine lokale Betäubung der Einstichstelle durch den Arzt, der anschließend die Kanüle für den Zugang an den Zugangspunkt heranführt.

Den Winkel, unter dem die Kanüle in den Zugangspunkt eingeführt werden muß, versucht der Arzt, basierend auf seiner Erfahrung, mehr oder weniger gut zu treffen. Dann wird die Kanüle teilweise in Richtung auf den Zielpunkt vorgeschoben. Nach Akquisition weitere Bilder wird die Position des Instrumentes kontrolliert und kann gegebenenfalls korrigiert werden. In Abhängigkeit von der Erfahrung des Therapeuten und dem Verlauf des Eingriffes werden die vorgenannten Schritte mehrfach interaktiv durchgeführt, bis der Zielpunkt im menschlichen Körper erreicht ist.

Die vorgenannten Beschreibung zur Durchführung eines mikrotherapeutischen Eingriffs unter Anwendung von bildgebenden Verfahren wie der Computertomographie oder der Kernspintomographie zeigt, dass ein Ansatzpunkt zur Kostenreduktion in der Möglichkeit eines gezielteren und damit zeitlich effizienteren mikrotherapeutischen Eingriffs besteht.

Dieser Ansatzpunkt ist eine effizientere Gestaltung der Arbeitsumgebung und der Arbeitsabläufe des Therapeuten sowie die Eröffnung der Automatisierung gewisser Arbeitsschritte. Die Bereitstellung einer Arbeitsumgebung, die zielgerichtere und verkürzte mikrotherapeutische Eingriffe zulässt, erlaubt kürzere und damit für den Patienten schonendere Eingriffe, die dann in ihrer Gesamtheit zu einer Kostenreduktion führen.

US 2003/0167061 offenbart eine Vorrichtung nach der Präambel von Anspruch 1.

Davon ausgehend, liegt der vorliegenden Erfindung die Aufgabe zugrunde, den Zugangspunkt als auch den genauen Zugangswinkel des interventionellen Instrumentes anzugeben und die Hinzuziehung der ungenauen anatomischen Referenzpunkte zu vermeiden. Gleichzeitig soll das Koordinatensystem von Computertomographen, als auch von Kernspintomographen für die Positionierung und Führung der interventionellen mikrotherapeutischen Eingriffe mitgenutzt werden, ohne weitere Koordinatensysteme zu integrieren. Auf diese Weise dienen der Tomograph und das von ihm erzeugte Koordinationssystem zugleich als Navigationssystem.

Diese Aufgabe wird ausgehend von einer Vorrichtung zum Positionieren von interventionellen Instrumenten innerhalb eines Untersuchungsraumes dadurch gelöst, dass gerichtete elektromagnetische Strahlung oder ein Zuführelement einen Zugangsbereich und die relative Ausrichtung des Instrumentes zur Erreichung des Zielbereiches, der in der Trajektorie liegt, markiert.

Von den beiden Alternativen ist die Verwendung einer gerichteten elektromagnetischen Strahlung zur Positionierung bevorzugt. Hierbei dient die Strahlung zur Ausrichtung eines interventionellen Instruments, dass vom behandelnden Arzt geführt wird. Die Strahlung dient dazu, den Zugangsbereich oder - punkt zu markieren und den Zielbereich bzw. -punkt zielgerichtet anzusteuern.

In der zweiten Alternative unter Verwendung eines Zuführelements steht die automatische Ausrichtung des interventionellen Instruments mit Hilfe des Zuführelements im Vordergrund. Dies erlaubt die automatische Ansteuerung des Zugangsbereiches oder -punkts und weiterhin die weitgehend automatische, aber auch manuelle, Ansteuerung des Zielbereichs bzw. -punkts.

Eine Kombination der beiden Alternativen, wie sie beispielsweise für die Steuerung eines Roboterarms mit Hilfe einer Laserquelle vorbeschlagen wurde, ist grundsätzlich möglich.

Die erfindungsgemäße Vorrichtung zum Positionieren von interventionellen Instrumenten erlaubt die positionsgenaue Markierung einer Trajektorie, die die Verlängerung der Geraden zwischen dem Zielbereich und dem Zugangsbereich beschreibt und insbesondere zwischen einem als Zugangspunkt und einem Zielpunkt.

Als gerichtete elektromagnetische Strahlungen kommt ein Strahl oder Strahlung im Bereich der Radiofrequenzen, der Mikrowellen, des Infratrots, auch des fernen oder des nahen Infrarotes, der UV-Bereich sowie, insbesondere der sichtbare oder Vis-bereich in Betracht. Zur genauen Markierung des Zugangsbereiches und der relativen Ausrichtung des Instrumentes besteht die Notwendigkeit, nicht streuende gerichtete elektromagnetische Strahlungen zu verwenden. Bevorzugt ist daher die Verwendung von Laserlicht, insbesondere von Laserlicht im Visbereich. Anwendungsbedingt wird ein Laser mit geringer Leistung verwendet, der zur Markierung der Trajektorie ausreichend ist. Die elektromagnetische Strahlung kann auch mit einer üblichen Optik, beispielsweise durch Fokussierung ausgerichtet werden.

Ein geeignetes Zuführelement umfasst einen Körper, beispielsweise einen Hohlkörper, insbesondere einen länglichen Hohlkörper mit zylindrischem Lumen. Dieses Lumen wird im Zugangsbereiche entsprechend ausgerichtet, dass ein interventionelles Instrument in ihm entlang einer Trajektorie vom Zugangsbereich in den Zielbereich geführt werden kann. Bevorzugt ist das Lumen an die Geometrie des interventionellen Instrumentes angepasst, um eine sehr genaue Positionierung des Instrumentes zu ermöglichen. In einer alternativen Ausführungsform umfasst das Zuführelement einen Körper mit wenigstens einer Führungsschiene, wobei die Führungsschiene in dem Untersuchungsraum so angeordnet wird, dass sie den Zugangsbereich und die relative Ausrichtung des Instrumentes zur Erreichung des Zielbereiches markiert. Ein Instrument kann dann manuell (vorzugsweise) oder Roboter gesteuert in dieser Führungsschiene bis in den Zielpunkt geschoben werden. Dabei ist es bevorzugt, dass der Körper außen auf seinem äußeren Umfang verschieden ausgeformte Führungsschienen aufweist, die jeweils passgenau für unterschiedliche interventionelle Instrumente ausgebildet sind. Auf diese Weise ist es nicht notwendig, das Führungselement während eines Eingriffs mit unterschiedlichen interventionellen Instrumenten auszuwechseln. Es versteht sich von selbst, dass das Zuführelement ein oder mehrere Lumen und/oder Führungsschienen, in Abhängigkeit vom Anforderungsprofil, aufweisen kann. Das Zuführelement bzw. der Hohlkörper kann gleichfalls ein üblicher Trokar oder Katether sein. Besteht das Zuführelement beispielsweise aus einem Lumen, insbesondere einer Führungshülse, so ist diese bevorzugt aufklappbar ausgeführt, so dass das interventionelle Instrument in jedem Verfahrensschritt frei gegeben werden kann, ohne dass die Führungshülse in ihrer Position verändert werden muß. Alternativ kann das Lumen beispielsweise einer Führungshülse seitliche Öffnungen aufweisen, um das Zuführelement in jedem Verfahrensschritt vom interventionellen Instrument lösen zu können. Bei Verwendung eines Zuführelementes mit Führungsschiene kann es bevorzugt sein, eine Führungsschiene mit einem bewegbaren Schlitten auszustatten, auf dem ein interventionelles Instrument mechanisch oder magnetisch fixiert werden kann. Auch in dieser Ausführungsvariante ist dann das interventionelle Instrument in jedem Verfahrensschritt von dem Zuführelement lösbar, ohne dass seine räumliche Ausrichtung verändert werden muß.

Die Vorrichtung zur Positionierung von interventionellen Instrumenten umfaßt ein Bild gebendes System, wobei das Bild gebende System ein MR-, ein CT-, ein Ultraschall-System oder ein anderes Schnittbildverfahren ist. Über die diagnostischen bildgebenden Systeme wird durch Aufzeichnung des Untersuchungsraumes der Zielbereich sowie der Zugangsbereich, insbesondere der Zugangspunkt und der Zielpunkt, sowie die Trajektorie ermittelt. Die Trajektorie definiert unmittelbar die relative Ausrichtung des Instrumentes im Zugangsbereich. Des Weiteren umfaßt die Vorrichtung einen Patiententisch der zumindest transversal bewegbar und/oder rotierbar ist. Zweckmäßigerweise ist das Material des Patiententisches und der gegebenenfalls aufgelegten Liege aus MR-kompatiblem Material oder röntgenschwachen Material ausgebildet.

Um die gerichtete elektromagnetische Strahlung oder das Zuführelement entsprechend den ermittelten Koordinaten einer Trajektorie ausrichten zu können, ist der Vorrichtung eine Trägervorrichtung zugeordnet. Dieser Trägervorrichtung ist die Strahlungsquelle selbst zugeordnet bzw. die Strahlung der Strahlungsquelle über beispielsweise eine Glasfaser oder das Zuführelement. Mittels der Trägervorrichtung kann die Strahlung und/oder das Zuführelement relativ zum Patiententisch bewegt und/oder fixiert werden.

Um eine Bewegung der Strahlung und/oder des Zuführelementes relativ zum Patiententisch auszuführen, kann die Trägervorrichtung als Brücke in Form eines Kreises oder Kreisabschnittes auf einem Radius um den Patiententisch herumgeführt sein, so dass die Strahlungsquelle oder das Zuführelement entlang der Trägervorrichtung auf einem Radius relativ zum Patiententisch bewegt werden können. Um weitere Freiheitsgrade in der relativen Ausrichtung der Strahlung und/oder des Zuführelementes im Hinblick auf den Patiententisch zu ermöglichen, können der Strahlungsquelle, der Zuleitung der Strahlung und/oder dem Zuführelement zusätzlich weitere Gelenke zugeordnet sein. Auf diese Weise ist einerseits eine Abstrahlung eines gerichteten Strahls ausgehend von einem variablen Punkt auf einer gedachten Kugeloberfläche möglich oder eine entsprechende Ausrichtung des Zuführelementes, ausgehend von diesem Punkt, möglich.

Die vorgenannte Anordnung der Vorrichtung mit einer Trägervorrichtung, die die Bewegung der Strahlungsquelle und/oder des Zuführelementes entlang eines äußeren Radius relativ zum Patiententisch erlaubt, ist einfach und kostengünstig herzustellen. Ohne eine Rotation des Patiententisches sind jedoch nur eingeschränkte Zugangswege möglich. Wird die Vorrichtung mit einem Kernspintomographen betrieben, ist nur ein rein transversaler Zugang zum Zielbereich bzw. Zielpunkt möglich und auch unter Verwendung der Computertomographie muß unter Beibehaltung des Gantry-Winkels gearbeitet werden.

Alternativ kann die Trägervorrichtung Abschnitte, Achsen oder Gelenke aufweisen, die eine transversale Bewegung und Rotation der Strahlung und/oder des Zuführelementes erlauben. Wird die Trägervorrichtung beispielsweise in Art eines Roboterarmes mit drei Abschnitten in Form von Roboterarmabschnitten ausgebildet, die ihrerseits über Achsen rotierbar miteinander verbunden sind, kann mit variablen Abständen zum Patiententisch gearbeitet werden. Zusätzlich besteht die Möglichkeit wenigstens einen Abschnitt des Roboterarmes sowohl für eine transversale Bewegung als auch für eine Rotation um die Achse, die senkrecht zum nächsten Abschnitt liegt, rotierbar zu gestalten.

Weitere zweckmäßige Ausgestaltungen können vorsehen, dass die Strahlungsquelle und/oder das Zuführelement selbst an einem Abschnitt derart bewegbar ist, dass der gerichtete Strahl, eine Führungsschiene oder ein Lumen des Zuführelementes von einem frei wählbaren Punkt auf einer Kugeloberfläche ausgehen. Zusätzlich besteht die Möglichkeit eine weitere Gelenkverbindung rotierbar durch die Verwendung eines Kugelgelenkes zu gestalten. Die vorgenannte Trägervorrichtung in Form dieses Roboterarmes, die über mindestens fünf Freiheitsgrade verfügt, erlaubt die freie Einstellung und Positionierung des gerichteten Strahls und/oder des Zuführelementes zur Abbildung bzw. Markierung des Zugangsweges.

Auf diese Weise kann bei beispielsweise Verwendung eines niederenergetischen Vis-Laserstrahls jede Position mit dem Laserstrahl angefahren und im Koordinatensystem des Untersuchungsraumes, und damit auch auf dem Patienten, abgebildet werden. Zur genauen Justage des Laserstrahls relativ zum Patiententisch im Koordinatensystem des Untersuchungsraumes kann ein Goniometer verwendet werden. Hierfür ist es bevorzugt, Schrittmotoren einzusetzen, die in der Lage sind sehr kleine Winkelbereiche von bis zu 10⁻⁴º abzufahren.

Mit der genannten Trägervorrichtung kann jeder Zugangsweg eines interventionellen Instrumentes mit dem Zuführelement angefahren und im Koordinatensystem des Untersuchungsraumes, und damit auch auf dem bzw. am Patienten, visualisiert werden. Zur genauen Justage des Zuführelementes relativ zum Patiententisch im Koordinatensystem des Untersuchungsraumes kann ein Goniometer verwendet werden. Hierfür ist es bevorzugt, Schrittmotoren einzusetzen, die in der Lage sind kleine Winkelbereiche von bis zu 10⁻⁴° abzufahren.

In Abstimmung mit der Trägervorrichtung kann die Ausrichtung des Patiententisches in ebenso kleinen Schritten rein transversal beispielsweise rauf/runter, vor/zurück oder recht/links durchgeführt werden. Eine geringe Neigung des Patiententisches kann ebenfalls erfolgen, doch sollte sie, um eine eigene Positionsveränderung des Patienten im Untersuchungsraum, und damit im Koordinatensystem der Vorrichtung zu vermeiden, nur zu einem geringen Ausmaß erfolgen.

Eine Kernidee der Erfindung ist die Begründung eines gemeinsamen Untersuchungsraumes und damit eines gemeinsamen Koordinatensystems sowohl des Bild gebenden Verfahrens als auch der der Trägervorrichtung zugeordneten Strahlungsquelle, des Zuführelementes und/oder einer Zuführvorrichtung.

Im Rahmen einer weiteren Optimierung des interventionellen Eingriffs besteht die Möglichkeit eine Nadel oder Kanüle über eine Zuführvorrichtung zur Positionierung des Instrumentes im Strahlengang der elektromagnetischen Strahlung vorzusehen. Durch die Verwendung dieser Zuführvorrichtung besteht die Möglichkeit auf andere Wellenlängenbereiche im elektromagnetischen Spektrum der Strahlung zurückzugreifen und eine andere Art der Markierung der korrekten Position des Instrumentes in der Trajektorie zu nutzen. Beispielsweise kann durch Reflexion (Totalreflexion, diffuse Reflexion) oder Absorption der Strahlung bei korrekter Positionierung des Instrumentes in der elektromagnetischen Strahlung ein optisches oder akustisches Signal erzeugt werden. Gleichfalls kann ein interventionelles Instrument beispielsweise eine Nadel oder Kanüle über diese Zuführvorrichtung zur Positionierung des Instrumentes im Lumen und/oder in einer Führungsschiene des Zuführelementes verwendet werden.

Im zweiten Schritt besteht die Möglichkeit, diese Zuführvorrichtung nicht nur zur Positionierung des Instrumentes in der elektromagnetischen Strahlung oder am Zuführelement zu nutzen, sondern auch direkt zum Einführen des Instrumentes entlang der Trajektorie vom Zugangsbereich in den Zielbereich hinein. Gleichfalls kann die Zuführvorrichtung zum Zuführen eines interventionellen Instrumentes in einem Untersuchungsraum zusammen mit einem Bild gebenden System unabhängig von der Vorrichtung zur Positionierung dazu genutzt werden, das Instrument entlang der Trajektorie vom Zugangsbereich in den Zielbereich hineinzuführen. Die Zuführvorrichtung kann beispielsweise ebenfalls, wie die Trägervorrichtung aus einem Roboterarm mit Abschnitten, Achsen oder Gelenken und einer Steuerung über Schrittmotoren bestehen. Bevorzugt verfügt die Zuführvorrichtung über bis zu sechs Freiheitsgrade, die durch die Rotation oder Translation von Abschnitten um Achsen und/oder Gelenke der Zuführeinrichtung möglich sind. Zweckmäßigerweise weist die Zuführvorrichtung einen Greifer zum Greifen des Instrumentes auf, wobei der Greifer selbst über Gelenke oder Achsen rotierbar mit der Zuführvorrichtung verbunden sein kann.

In einer zweckmäßigen Weiterbildung ist die Vorrichtung zusätzlich mit einem Detektor, beispielsweise einem CCD-Detektor versehen, der über die Messung der Reflexion oder Absorption eine Abstandsbestimmung des interventionellen Instrumentes zur elektromagnetischen Strahlungsquelle bzw. zu Berechnungen der Eindringtiefe des Instrumentes erlaubt. Dies kann beispielsweise auch durch Ermittlung der Koordinaten des Instrumentes im gemeinsamen Untersuchungsraum geschehen. Alternativ kann eine elektrische Widerstandsmessung zur Bestimmung der Eindringtiefe der interventionellen Instrumente oder ein Einsatz eines Markierungszählers vorgesehen werden.

Die Vorrichtung kann zusätzlich einen Computer, einen Schrittmotor, der beispielsweise der Träger- und/oder der Zuführvorrichtung zugeordnet ist, eine Steuereinheit, eine Anzeigeneinheit, eine Rekonstruktionseinheit, einen Detektor sowie Software zur Auswertung und Steuerung des Bild gebenden Systems, die Trägervorrichtung und/oder die Zuführvorrichtung sowie Schnittstellen umfassen.

Bei einem darüber hinaus beschriebenen Verfahren wird die Positionierung eines interventionellen Instrumentes innerhalb eines Untersuchungsraumes dadurch ermöglicht, dass die Markierung zur Positionierung des Instrumentes im Zugangsbereich, als auch die relative Ausrichtung des Instrumentes durch den Strahlengang eines gerichteten elektromagnetischen Strahls und/oder eines Zuführelementes erfolgt. Dabei liegt der Zielbereich in der Trajektorie des elektromagnetischen Strahls und/oder in der Trajektorie eines Lumen oder einer Führungsschiene des Zuführelementes. Die Ermittlung oder Berechnung des Zugangsbereiches, des Zielbereiches, insbesondere des Zugangspunktes und des Zielpunktes, sowie der Trajektorie, erfolgt nach Maßgabe von diagnostischen Bilddaten, die über ein bildgebendes Verfahren ermittelt werden. Erfindungsgemäß wird das Koordinatensystem, aus dem bildgebenden Verfahren sowohl von Computertomographen als auch von Kernspintomographen oder anderen schnittbildgebenden Verfahren, für die Steuerung und Positionierung der elektromagnetischen Strahlung und/oder des Zuführelementes innerhalb des Untersuchungsraumes genutzt. Es wird kein zweites Koordinatensystem benötigt, das mit dem des bildgebenden Verfahrens abgestimmt (koordiniert) werden müsste.

Das Zuführelement kann zur positionsgenauen Ausrichtung von interventionellen Instrumenten genutzt werden. Dazu ist das Instrument vorzugsweise in jedem Verfahrensschritt von dem Zuführelement lösbar. Das Zuführelement kann beispielsweise klappbar oder mit einer seitlichen Öffnung entlang der Längserstreckung des Zuführelementes ausgebildet sein. Die Führungsschienen können bewegliche Schlitten aufweisen, auf denen ein interventionelles Instrument mechanisch oder magnetisch reversibel fixiert ist. Aus diese Weise ist ein interventionelles Instrument in jedem Verfahrensschritt von dem Zuführelement lösbar, ohne dass die räumliche Ausrichtung des Instrumentes verändert wird.

In einer zweckmäßigen Ausgestaltung des Verfahrens besteht die Möglichkeit, den Abstand zwischen einem Punkt auf dem interventionellen Instrument, bevorzugt den Abstand zwischen einem zuvor genau definierten Punkt am Ende des Instrumentes und der Strahlungsquelle oder einem zuvor bestimmten Punkt im gemeinsamen Koordinatensystem des Untersuchungsraumes zu messen. Die Messung kann durch die Absorption oder Reflexion der gestreuten Strahlung erfolgen und über einen Detektor gemessen werden. Alternativ kann die Abstandsmessung eines Punktes auf dem interventionellen Instrument über eine Markierung, beispielsweise mittels eines Röntgenmarkers, auf dem Instrument erfolgen. Bei Berücksichtigung der Geometrie des Instrumentes, beispielsweise der Länge der Nadel, ist die Berechnung der Einstichtiefe möglich. Üblicherweise wird die Position des interventionellen Instrumentes innerhalb des Untersuchungsraumes mittels eines Kernspintomographen oder Computertomographens ermittelt. Auch ein Markierungszähler oder ein Apparat zur Bestimmung des elektrischen Widerstandes können zur Ermittlung der Einstichtiefe eingesetzt werden.

Zusammen mit der Vorrichtung zum Positionieren von interventionellen Instrumenten werden zweckmäßig speziell für diese Anwendung konzipierte Instrumente eingesetzt. Ein Ansatzpunkt besteht in der Auswahl der Materialien zur Herstellung der interventionellen Instrumente. Für eine Verwendung mit MR basierten bildgebenden Verfahren sollten biokompatible Materialien zur Anwendung kommen, die kein Signal verursachen und deren Suszeptibilitätskonstante ähnlich der von Gewebe ist, um keine Suszeptibilitätsartefakte auszulösen. In MR-Verfahren werden üblicherweise homogene Magnetfelder verwendet. Bei Anwendungen, die auf der Nutzung von Röntgenstrahlung beruhen sind biokompatible Materialien, die eine von Gewebe verschiedene Röngenstreuung aufweisen, zweckmäßig. Weitere Ansatzpunkte bietet die spezielle Ausgestaltung der interventionellen Instrumente. Um die genaue Position nicht nur im gemeinsamen Koordinatensystem abbilden zu können, sondern auch die Eindringtiefe der Instrumente in das Objekt visuell für den Anwender wahrnehmbar zugestalten, können an den Instrumenten, insbesondere bei Nadeln, Kanülen, Trokaren, Biopsienadeln oder Elektrolysnadeln entsprechende Markierungen vorgesehen sein. An den Markierungen ist dann eine unmittelbare Bestimmung der Eindringtiefe möglich. Denkbar ist eine Skalierung und/oder Farbgebung. Bei CT-geführten Verfahren kann es sich als vorteilhaft erweisen als Markierungen zur Bestimmung der Eindingtiefe alternativ oder zusätzlich Röntgenmarker vorzusehen. Eine weitere Möglichkeit zur Bestimmung der Eindringtiefe, insbesondere über ein Referenzsystem, besteht darin, dass Bereiche der interventionellen Instrumente so ausgebildet sind, dass ein Teil der elektromagnetischen Strahlung definiert durch sie hindurchstrahlen kann oder auch Bereiche so ausgebildet sind, dass ein Teil der Strahlung diffus oder als Totalreflexion auf einen Detektor gelenkt wird. Über die Auswertung des Strahlengangs kann so die korrekte Position und/oder die Eindringtiefe des Instrumentes bestimmt werden.

Wie bereits ausgeführt wird erfindungsgemäß das vorhandene Koordinatensystem, eines bildgebenden Verfahrens beispielsweise eines Computertomographen, eines Kernspintomographen oder eines anderen bildgebenden Systems, für die Steuerung und Ausrichtung von elektromagnetischer Strahlung und/oder des Zuführelementes innerhalb des Untersuchungsraumes genutzt. Das dazu benötigte Computerprogramm kann beispielsweise auf bestehende Hardware der bildgebenden Systeme implementiert werden. Das Computerprogramm ermöglicht die Steuerung der erfindungsgemäßen Positioniervorrichtung. Das erfindungsgemäße Computerprogramm berechnet aus Volumenbilddaten eines Untersuchungsbereiches mögliche Positionsdaten für Zugangsbereiche und Zielbereiche, bevorzugt Zugangspunkte und Zielpunkte, sowie die Trajektorie und steuert über eine Steuereinheit eine Trägervorrichtung mit einer zugeordneten Strahlungsquelle, so dass der gerichtete elektromagnetische Strahl in einer der berechneten Trajektorien liegt. Auf entsprechende Weise steuert eine Steuereinheit auch eine Trägervorrichtung, der beispielsweise zusätzlich oder alleine das Zuführelement zugeordnet ist. Ein Lumen oder eine Führungsschiene des Zuführelementes wird von der Trägervorrichtung so ausgerichtet, dass der Zugangsbereich und der Zielbereich, der in einer der berechneten Trajektorien liegt, aufgezeigt wird und in einer der berechneten Trajektorien liegt. In einer besonders bevorzugten Variante ist das Computerprogramm entsprechend programmiert, dass der Untersuchungsbereich, der Zugangsbereich, der Zielbereich und die Trajektorie in einem gemeinsamen Koordinatensystem liegen, wobei die relative Ausrichtung des Patiententisches zur Strahlung und/oder zum Zuführelement zueinander berechnet wird und über eine Steuereinheit ausrichtbar ist.

Das Koordinatensystem stimmt mit dem bereits vorhanden Koordinatensystem eines bildgebenden Systems überein. Der Vorteil der Verwendung eines gemeinsamen Koordinatensystems besteht in der Möglichkeit der genauen und einheitlichen Definition von Punkten, wie dem Ziel- und Zugangspunkt sowie der Trajektorie, wobei gleichzeitig auf die ungenauen anatomischen Referenzpunkte der Verfahren gemäß dem Stand der Technik, verzichtet wird. Rechenleistung und -zeit sowie die unvermeidbare Ungenauigkeit bei der Anpassung getrennter Koordinatensysteme aneinander wird vermieden. Gleichzeitig wird eine Überwachung des Eingriffs durch das bildgebende Verfahren und eine Nachjustierung ermöglicht.

In einer zweckmäßigen Variante steuert das Computerprogramm eine Steuereinheit einer Zuführvorrichtung an, und bringt ein interventionelles Instrument in die Trajektorie bzw. in den gerichteten Strahlengang einer Strahlenquelle oder in ein Lumen/Führungsschiene eines Zuführelementes ein, wobei ein Ende des Instrumentes im Zugangsbereich positioniert wird und in einem zweiten Schritt die Zuführung des Instrumentes über den Zugangsbereich entlang der Trajektorie in den Zielbereich gesteuert werden kann. Zusätzlich besteht die Möglichkeit das Computerprogramm so zu modifizieren, dass es über eine Auswertung von Volumendaten eines Untersuchungsbereiches die Positionen des Instrumentes bestimmt oder aus Messdaten eines Detektors Abstandsdaten des Instrumentes ermittelt.

Dieses Computerprogramm kann vorteilhafterweise den Benutzern von herkömmlichen bildgebenden Diagnosegeräten auf geeigneten Datenträgern, wie beispielsweise CD-Rom, DVDs zur Verfügung gestellt werden, oder es kann auch über ein öffentliches Datennetz (Internet) zum Herunterladen bereitgehalten werden.

Ausführungsbeispiele der Erfindung werden im Folgenden anhand von Abbildungen erläutert. Es zeigen:
- Figur 1a: die erfindungsgemäße Markierung des Zugangsbereiches mittels gerichteter elektromagnetischer Strahlung und
- Figur 1b: die relative Ausrichtung des Instrumentes in der Trajektorie, um den Zielbereich zu erreichen;
- Figur 1c: die erfindungsgemäße Markierung des Zugangsbereiches mittels eines Zuführelementes und
- Figur 1d: ein alternatives Zuführelement;
- Figur 2: einen Patienten im Untersuchungsraum auf einer Liege eines Patiententisches vor der Gantry mit einem durch einen Laserstrahl definierten Zugangsweg;
- Figur 3: zeigt einen mit einer Tomographieeinheit verbundenen Laser mit zwei Bewegungsfreiheitsgraden;
- Figur 4a: zeigt eine Trägervorrichtung in einer Ausführung als Roboterarm mit fünf Bewegungsfreiheitsgraden unter Verwendung von gerichteter elektromagnetischer Strahlung;
- Figur 4b: zeigt eine Trägervorrichtung in einer Ausführung als Roboterarm mit fünf Bewegungsfreiheitsgraden unter Verwendung eines Zuführelementes;
- Figur 5: zeigt eine Träger- und eine Zuführvorrichtung mit fünf Bewegungsfreiheitsgraden.

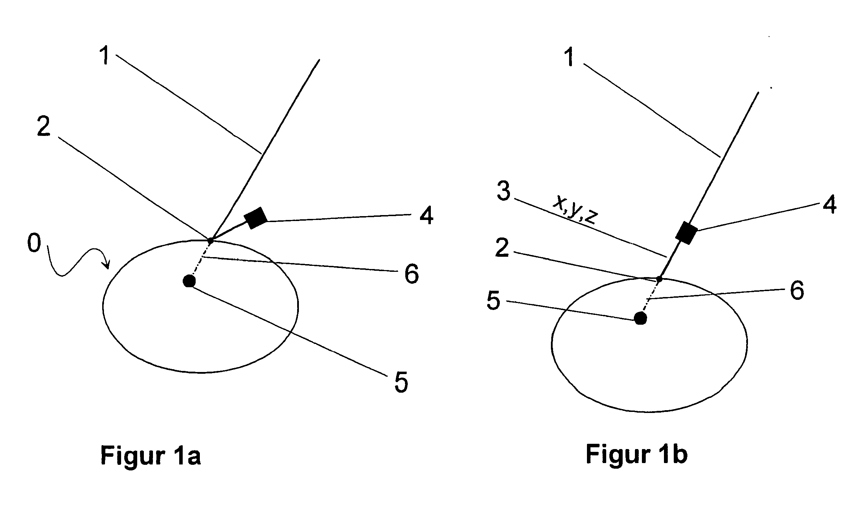

Die Figur 1a zeigt ein Objekt 0 bzw. einen Patienten mit einer Projektion eines elektromagnetischen Strahls 1, der den Zugangspunkt 2 auf der Oberfläche des Objektes markiert und in dessen Trajektorie 6 der Zielbereich 5 liegt. Das interventionelle Instrument 4 liegt mit einem Ende im Zugangspunkt 2, der durch die elektromagnetische Strahlung 1 markiert wird. Die Ausrichtung des interventionelle Instrument 4 relativ zur elektromagnetischen Strahlung 1 ist so, dass die elektromagnetische Strahlung in der Längsachse des interventionellen Instrumentes 4 liegt. Die relative Ausrichtung 3 des interventionellen Instrumentes 4 erfolgt also mit Hilfe der elektromagnetischen Strahlung 1, siehe Figur 1b. Als elektromagnetische Strahlung eignet sich besonders ein Laserstrahl mit einer Wellenlänge im Visbereich. In Abhängigkeit von der Ausführungsform des interventionellen Instrumentes kann die korrekte Ausrichtung des Instrumentes in der elektromagnetischen Strahlung anhand äußerer Markierung auf dem Instrument registriert werden. Bei Verwendung einer Kanüle, kann die richtige Ausrichtung über das Auftreffen des Laserstrahls auf der Oberfläche des Objektes registriert werden. Dieser Laserstrahl strahlt an einem Ende in die Kanüle ein und tritt, bei korrekter Ausrichtung der Kanüle, auf der entgegengesetzten Seite aus und markiert den Zugangspunkt auf der Hautoberfläche. Das Instrument 4 kann auch seinem der Strahlungsquelle zugewandten Ende einen Spiegel aufweisen, dessen Reflexionen über einen Detektor zur Ausrichtung des Instruments 4 verwandt werden.

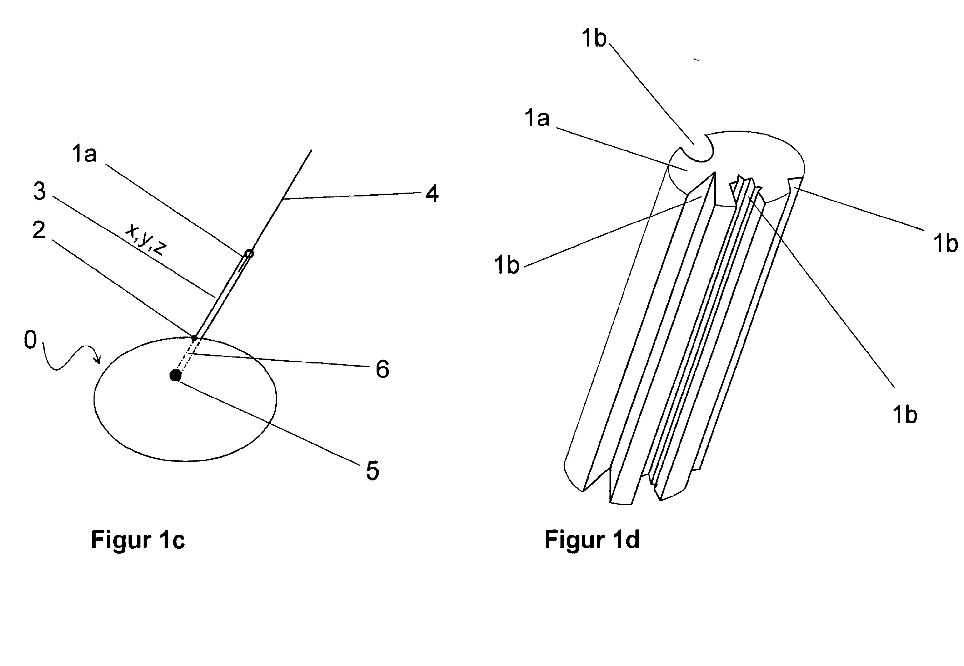

Die Figur 1c zeigt ein Objekt 0 bzw. einen Patienten mit einem zugeordneten Zuführelement 1a, dass den Zugangspunkt 2 auf der Oberfläche des Objektes markiert sowie den Zielbereich 5, der in einer der Trajektorien 6 des Zuführelementes 1a liegt. Das Zuführelement gemäß Figur 1c besteht aus einem Hohlkörper mit einem zylindrischen Hohlraum bzw. Lumen, wobei durch die relative Ausrichtung 3 des Lumen im Zugangsbereich 2, ein durch dieses Lumen hindurch geführtes interventionelles Instrument 4, durch Verschieben entlang wenigstens einer der Trajektorien 6 in den Zielbereich 5 hineingeführt werden kann.

Die Figur 1d zeigt eine alternative Ausführungsform eines Zuführelementes 1a, umfassend einen Körper mit verschieden ausgestalteten Führungsschienen 1b. Die Führungsschienen 1b sind entsprechend geometrisch an denkbare interventionelle Instrumente (nicht abgebildet) angepasst. Eine Führungsschiene 1b kann beispielsweise im Zugangsbereich gemäß Figur 1c, alternativ zum dort gezeigten Hohlkörper relativ auf dem Objekt ausgerichtet werden, so dass ein interventionelles Instrument 4 entlang einer Führungsschiene 1b über den Zugangsbereich 2 in den Zielbereich 5, der in der Trajektorie 6 einer Führungsschiene 1b liegt, hinein geführt werden.

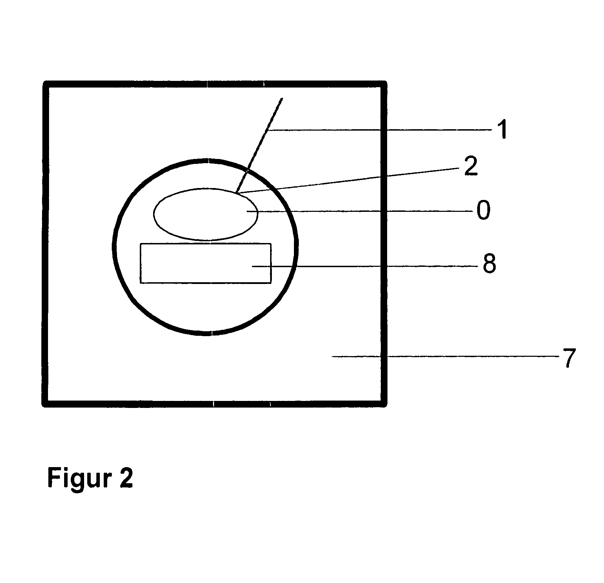

In der Figur 2 ist ein Objekt 0 bzw. ein Patient dargestellt, der vor der Gantry des Bild gebenden Systems 7 auf einem Patiententisch 8 liegt. Die elektromagnetische Strahlung 1, in diesem Fall ein niederenergetischer Laserstrahl markiert den Zugangspunkt 2 auf der Oberfläche des Objektes 0.

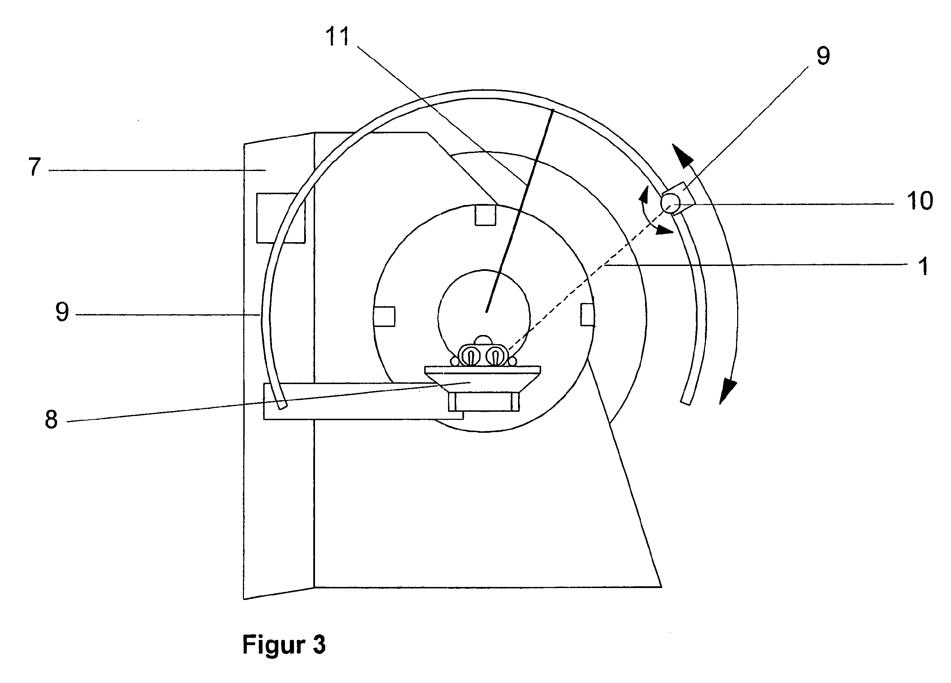

Die Figur 3 zeigt ein Ausführungsbeispiel der erfindungsgemäßen Vorrichtung mit einer Tomographieeinheit 7, die mit einem Laser 1 versehen ist. Die Strahlungsquelle 10 ist an einer Trägervorrichtung 9 angeordnet, wobei die Strahlungsquelle 10 entlang des Radius 11 relativ zum Patiententisch 8 bewegt werden kann. Ein weiterer Freiheitsgrad der Strahlungsquelle 10 wird durch die Möglichkeit der Rotation der Strahlungsquelle eröffnet. Weitere Freiheitsgrade sind durch die transversale Bewegung und Rotation des Patiententisches 8 erreichbar. In der Figur 3 ist ein System abgebildet, über das relativ einfach ein bestehendes System wie ein MRT oder ein CT-System über die Erweiterung mit der Trägervorrichtung 9 ausbaubar ist. Die in der Figur 3 dargestellte Geometrie erlaubt nur definierte Zugangswege. So ist der Zugangsweg für eine Anwendung mit MRT nur transversal und unter Verwendung eines CT-Systems ausschließlich unter Beibehaltung des Gantry-Winkels möglich.

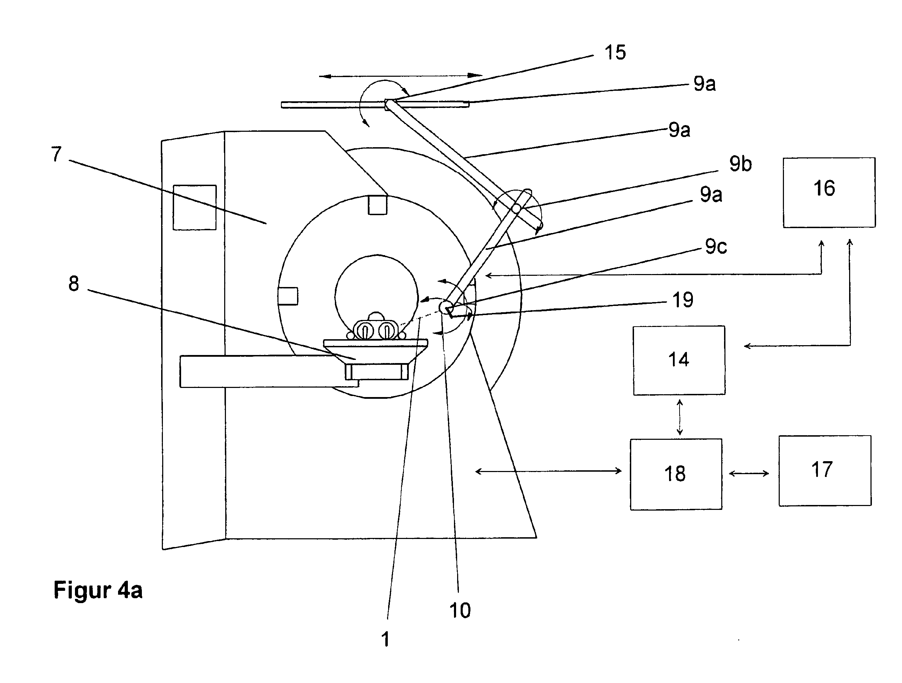

Eine Vorrichtung zum Positionieren von interventionellen Instrumenten innerhalb eines Untersuchungsraumes, bei der die Trägervorrichtung 9 in Form eines Roboterarms ausgebildet ist, ist in Figur 4a dargestellt. Das Bild gebende System 7 ist ein MR- oder CT-System. In der gezeigten Ausführungsvariante ist es vorgesehen, dass die Strahlungsquelle 10 frei rotierbar an einem Abschnitt 9a der Trägervorrichtung 9 angeordnet ist und über weitere Achsen 9b und Gelenke 9c sowohl transversale Bewegungen als auch Rotationen ausführen kann. Die in Figur 4a abgebildete Anordnung verleiht der Trägervorrichtung 9 fünf eigene Freiheitsgrade. An den Achsen 9b oder Gelenken 9c sind Schrittmotoren 15 vorgesehen, die eine Winkeleinstellung im Bereich von 10⁻⁴° Schritten erlauben. Diese Schrittmotoren 15 werden über die Steuereinheit 16 angesteuert. Die Eindringtiefe des interventionellen Instrumentes in das Objekt ist über eine Auswertung laufender Volumenbilddaten des Untersuchungsraumes möglich.

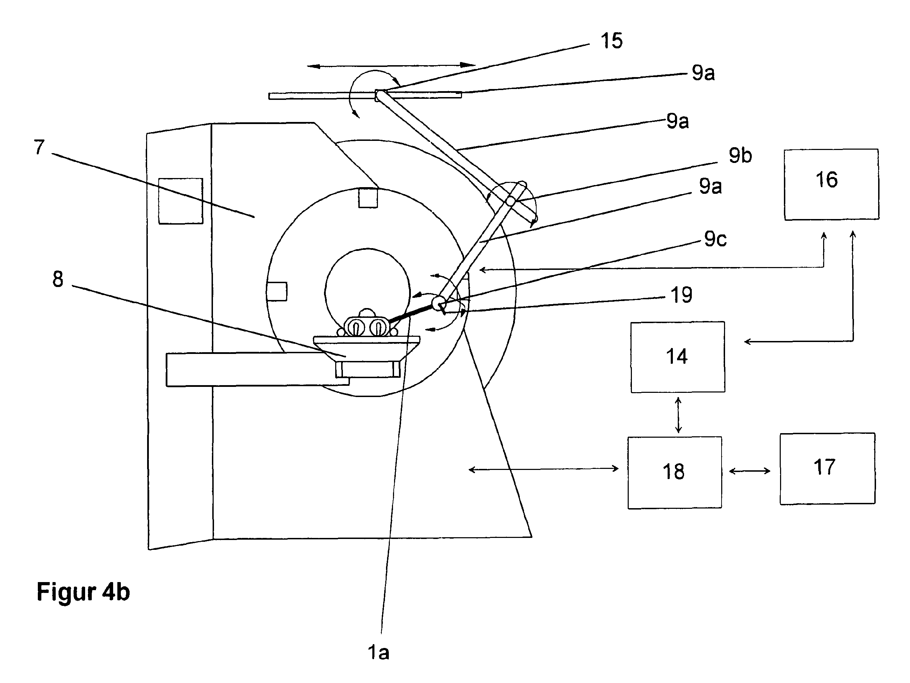

Eine alternative Vorrichtung zum Positionieren von interventionellen Instrumenten innerhalb eines Untersuchungsraumes, zeigt die Figur 4b, bei der die Trägervorrichtung 9 in Form eines Roboterarms ausgebildet ist. Das bildgebende System 7 ist ein MR-, CT-System oder ein anderes schnittbildgebendes Verfahren. In der gezeigten Ausführungsvariante ist es vorgesehen, dass ein Zuführelement 1a frei rotierbar an einem Abschnitt 9a der Trägervorrichtung 9 angeordnet ist und über weitere Achsen 9b und Gelenke 9c sowohl transversale Bewegung als auch Rotationen ausführen kann. Die in Figur 4b abgebildete Anordnung verleiht der Trägervorrichtung 9 fünf eigene Freiheitsgrade. An den Achsen 9b oder Gelenken 9c sind Schrittmotoren 15 vorgesehen, die eine Winkeleinstellung im Bereich von 10⁻⁴ erlauben. Diese Schrittmotoren werden über die Steuereinheit 16 angesteuert. Die Bestimmung der Eindringtiefe des interventionellen Instrumentes in das Objekt ist über die vorgenannten Methoden möglich.

Sofern das Zuführelement 1a aus einem Lumen, insbesondere aus einer Führungshülse besteht, so ist es bevorzugt, dass diese entlang ihrer Längserstreckung zu öffnen ist, um das interventionelle Instrument in jedem Verfahrensschritt, beispielsweise nach dem ersten Einstich im Zugangsbereich 2, frei gegeben werden kann. Alternativ kann das Lumen des Zuführelementes 1a seitliche Öffnungen in Längserstreckung des Zuführelementes aufweisen, um das Zuführelement 1a in jedem Verfahrensschritt von dem interventionellen Instrument loslösen zu können. Werden Zuführelemente 1a mit Führungsschienen verwendet, so kann es bevorzugt sein, dass eine Führungs einen bewegbaren Schlitten zur Positionierung und/oder Zuführung eines interventionellen Instrumentes aufweist. Auf diesem kann ein interventionelles Instrument mechanisch oder magnetisch fixiert werden. Auch in dieser Ausführungsvariante ist das interventionelle Instrument in jedem Verfahrensschritt von dem Zuführelement lösbar, ohne dass seine räumliche Ausrichtung verändert werden muß.

Alternativ ist eine Abstandsmessung, die die Eindringtiefe des interventionellen Instrumentes in das Objekt angibt, möglich. In der Ausführungsvariante gemäß Figur 4a ist ein Detektor 19 der Trägervorrichtung 9 zugeordnet, der die Reflexion der elektromagnetischen Strahlung mißt, die dann über einen Computer ausgewertet, die Eindringtiefe in das Objekt oder den Abstand zwischen der Strahlungsquelle und einem Punkt auf dem interventionellen Instrument angibt. Die Bild gebende Einheit umfasst einen Computer 14, der einer Rekonstruktionseinheit 18 zugeordnet ist. In der Rekonstruktionseinheit werden entweder aus Röntgensignalen Schichtbilder rekonstruiert oder aus den Relaxationszeiten der Magnetresonanzspektroskopie Schichtbilder bzw. Volumendaten rekonstruiert. In der Anzeigeneinheit 17 wird eine zwei- bzw. dreidimensionale Ansicht des Untersuchungsraumes, insbesondere des Koordinatensystems des Untersuchungsraumes dargestellt. In der Anzeigeneinheit 17 wird insbesondere auch die Trajektorie 6 im Koordinatensystem des gemeinsam Untersuchungsraumes sowie die gerichtete elektromagnetische Strahlung 1, der Zugangsbereich 2, die relative Ausrichtung 3 des Instrumentes 4 und der Zielbereich 5 visuell dargestellt.

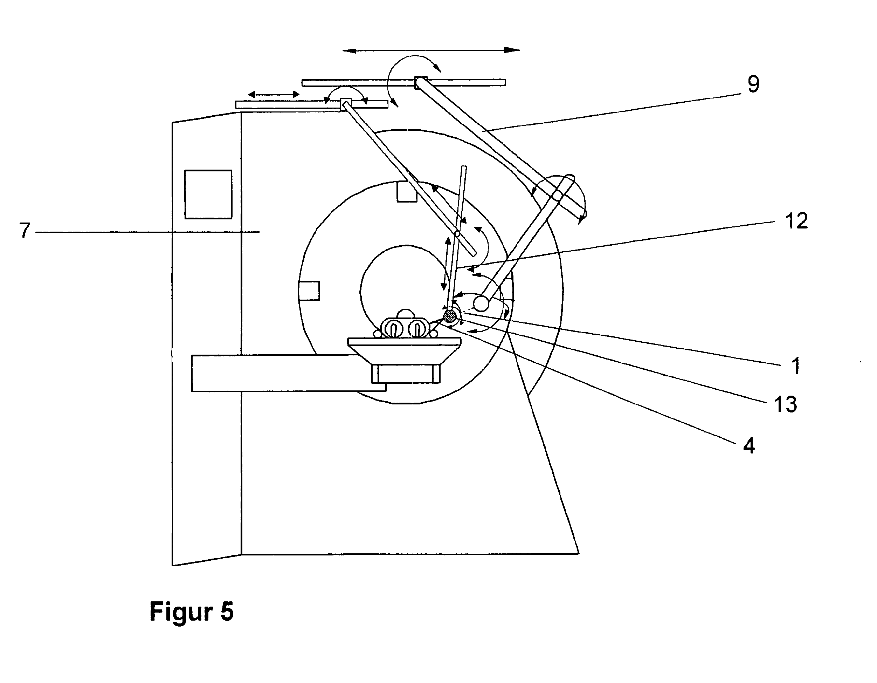

Die Figur 5 zeigt die Vorrichtung zum Positionieren von interventionellen Instrumenten mit einer Trägervorrichtung 9 und einer Zuführvorrichtung 12. Die Zuführvorrichtung 12 verfügt in dieser Ausführungsvariante über einen Greifer 13 und kann transversale Bewegungen und Rotationen um sieben Freiheitsgrade ausführen. Die Zuführvorrichtung 12 hat entsprechend der Trägervorrichtung 9 Abschnitte, Achsen und Gelenke. Außerdem verfügt sie über Schrittmotoren, um bestimmte Positionen im Koordinatensystem anfahren zu können. Die Schrittmotoren werden entsprechend über eine Steuervorrichtung in Abstimmung auf die Trajektorie bzw. den gerichteten elektromagnetischen Strahl im gemeinsamen Koordinatensystem des Untersuchungsraumes abgestimmt und angesteuert. Die Funktion der Zuführvorrichtung ist die Positionierung des Instrumentes in der elektromagnetischen Strahlung und die Positionierung des einzuführenden Endes des Instrumentes im Zugangspunkt auf der Hautoberfläche. In einem weiteren Schritt kann die Zuführvorrichtung auch zum Einführen des Instrumentes entlang der Trajektorie zwischen Zugangsbereich und Zielbereich genutzt werden. Durch die Verwendung der Zuführvorrichtung zusammen mit der Trägervorrichtung erlaubt eine doppelte Kontrolle der Positionsgenauigkeit beim Einführen des interventionellen Instrumentes. Insgesamt erlaubt die erfindungsgemäße Vorrichtung zum Positionieren von interventionellen Instrumentes innerhalb eines Untersuchungsraumes, unter Verwendung eines gemeinsamen Koordinatensysteme eine schnellere und zielgerichtetere Positionierung des interventionellen Instrumentes auf der Oberfläche eines Objektes, insbesondere auf der Haut eines Patienten, sowie die genaue relative Ausrichtung des Instrumentes. Gleichfalls ist eine rein visuelle Kontrolle der positionsgenauen Einführung des interventionellen Instrumentes in den Körper des Patienten durch die Abbildung des Laserstrahls auf einen Punkt des interventionellen Instrumentes möglich. Insgesamt verkürzt die Vorrichtung zum Positionieren von interventionellen Instrumenten die Behandlungsdauer und verbessert deutlich die Treffsicherheit des Therapeuten und bedingt so einen schonenderen mikrotherapeutischen Eingriff am Patienten.

## Patentansprüche

1. Vorrichtung zum Positionieren von interventionellen Instrumenten innerhalb eines Untersuchungsraumes, mit einem transversal bewegbaren und/oder rotierbaren Patiententisch (8), einem bildgebenden System zur Aufnahme von diagnostischen Bilddaten des Untersuchungsraumes, und mit einer Strahlungsquelle (10), die gerichtete elektromagnetische Strahlung erzeugt, oder einem zufahrelement (1a) wobei mittels der Strahlungsquelle (10) oder mittels des Zuführelements (1a) ein Zugangspunkt (2) und die relative Ausrichtung (3) eines Instrumentes (4) zur Erreichung eines Zielpunktes (5), der in einer Trajektorie (6) des Instrumentes (4) liegt, markierbar sind, wobei
der Strahlungsquelle (10) oder dem Zuführelement (1a) eine Trägervorrichtung (9) zugeordnet ist, mittels welcher die Strahlungsquelle (10) oder das Zuführelement relativ zu dem Patiententisch (8) positionierbar sind, wobei die Vorrichtung eingerichtet ist zur Ermittlung oder Berechnung des Zugangspunktes (2), des Zielpunktes (5) und der Trajektorie (6) nach Maßgabe der diagnostischen Bilddaten und zur entsprechenden Steuerung der Trägervorrichtung (9) mittels einer Steuereinheit (16), **dadurch gekennzeichnet, dass** die Positionierung der Trägervorrichtung (9) in dem Koordinatensystem des bildgebenden Systems erfolgt, und zwar ohne Abstimmung mit einem zweiten Koordinatensystem der Trägervorrichtung (9), der Strahlungsquelle (10) oder des Zuführelementes (1a).

2. Vorrichtung nach Anspruch 1 , **dadurch gekennzeichnet, dass** die gerichtete elektromagnetische Strahlung Laserlicht ist.

3. Vorrichtung nach Anspruch 1 , **dadurch gekennzeichnet, dass** das Zuführelement ein Hohlkörper oder ein Körper mit einer Führungsschiene (1 b) ist.

4. Vorrichtung nach einem der Ansprüche 1 bis 3, **dadurch gekennzeichnet, dass** das bildgebende System ein MR-, CT-, oder Ultraschall-System ist.

5. Vorrichtung nach einem der Ansprüche 1 bis 4, **dadurch gekennzeichnet, dass** der Patiententisch (8) transversal bewegbar und/oder rotierbar ist.

6. Vorrichtung nach einem der Ansprüche 1 bis 5, **dadurch gekennzeichnet, dass** die Trägervorrichtung (9) die Bewegung der Strahlungsquelle (10) oder des Zuführelementes (1a) entlang eines Radius (11) relativ zum Patiententisch (8) erlaubt.

7. Vorrichtung nach einem der Ansprüche 1 bis 6, **dadurch gekennzeichnet, dass** die Trägervorrichtung (9) Abschnitte (9a), Achsen (9b) oder Gelenke (9c) aufweist, die eine transversale Bewegung und Rotation der Strahlung der Strahlungsquelle (10) oder des Zuführelementes (1a) erlaubt.

8. Vorrichtung, insbesondere nach einem der Ansprüche 1 bis 7, **dadurch gekennzeichnet, dass** ihr eine Zuführvorrichtung (12) zur Positionierung des Instrumentes (4) in der gerichteten elektromagnetischen Strahlung, zur Positionierung am Zuführelement (1a) und/oder zum Einführen des Instruments entlang der Trajektorie (6) zwischen Zugangsbereich (2) und Zielbereich (5) zugeordnet ist.

9. Vorrichtung nach Anspruch 7 oder 8, **dadurch gekennzeichnet, dass** die Zuführvorrichtung (12) einen Greifer (13) aufweist.

## Claims

1. Apparatus for positioning interventional instruments within an examination room, comprising a transversely movable and/or rotatable patient table (8), an imaging system for recording diagnostic image data of the examination room, and a radiation source (10) which produces directed electromagnetic radiation or a feed element (1a), wherein an access point (2) and the relative orientation (3) of an instrument (4) can be marked by means of the radiation source (10) or by means of the feed element (1a) to reach a target point which is in a trajectory (6) of the instrument (4), wherein associated with the radiation source (10) or the feed element (1a) is a carrier device (9), by means of which the radiation source (10) or the feed element can be positioned relative to the patient table (8), wherein the apparatus is adapted to ascertain or calculate the access point (2), the target point (5) and the trajectory (6) in accordance with the diagnostic image data and to correspondingly control the carrier device (9) by means of a control unit (16), **characterised in that** positioning of the carrier device (9) is effected in the co-ordinate system of the imaging system, more specifically without co-ordination with a second co-ordinate system of the carrier device (9), the radiation source (10) or the feed element (1a).

2. Apparatus according to claim 1 **characterised in that** the directed electromagnetic radiation is laser light.

3. Apparatus according to claim 1 **characterised in that** the feed element is a hollow body or a hollow body having a guide rail (1b).

4. Apparatus according to one of claims 1 to 3 **characterised in that** the imaging system is an MR, CT or ultrasound system.

5. Apparatus according to one of claims 1 to 4 **characterised in that** the patient table (8) is transversely movable and/or rotatable.

6. Apparatus according to one of claims 1 to 5 **characterised in that** the carrier device (9) allows the movement of the radiation source (10) or the feed element (1a) along a radius (11) relative to the patient table (8).

7. Apparatus according to one of claims 1 to 6 **characterised in that** the carrier device (9) has portions (9a), shafts (9b) or joints (9c), which allows a transverse movement and rotation of the radiation of the radiation source (10) or the feed element (1a).

8. Apparatus, in particular according to one of claims 1 to 7, **characterised in that** associated therewith is a feed device (12) for positioning the instrument (4) in the directed electromagnetic radiation, for positioning on the feed element (1a) and/or for introducing the instrument along the trajectory (6) between the access region (2) and the target region (5).

9. Apparatus according to claim 7 or claim 8 **characterised in that** the feed device (12) has a gripping means (13).

## Revendications

1. Dispositif de positionnement d'instruments pour intervention à l'intérieur d'une salle d'examen, avec une table de patient (8) déplaçable transversalement et/ou tournante, un système d'imagerie pour la réception de données d'image diagnostics de la salle d'examen, et avec une source de rayonnement (10) qui produit le rayonnement électromagnétique dirigé, ou d'un élément d'amenée (1a), où peuvent être marqués au moyen de la source de rayonnement (10) ou au moyen de l'élément d'amenée (1a) un point d'accès (2) et l'orientation relative (3) d'un instrument (4) pour l'atteinte d'un point visé (5) qui se situe sur une trajectoire (6) de l'instrument (4), où est associé à la source de rayonnement (10) ou à l'élément d'amenée (1a) un dispositif de support (9) au moyen duquel la source de rayonnement (10) ou l'élément d'amenée peuvent être positionnés relativement à la table de patient (8), où le dispositif est agencé pour la détermination ou le calcul du point d'accès (2), du point visé (5) et de la trajectoire (6) dans le cadre des données d'image diagnostic et pour la commande correspondante du dispositif de support (9) au moyen d'une unité de commande (16), **caractérisé en ce que** le positionnement du dispositif de support (9) a lieu dans le système de coordonnées du système d'imagerie, à savoir sans coordination avec un deuxième système de coordonnées du dispositif de support (9), de la source de rayonnement (10) ou de l'élément d'amenée (1a).

2. Dispositif selon la revendication 1, **caractérisé en ce que** le rayonnement électromagnétique dirigé est la lumière laser.

3. Dispositif selon la revendication 1, **caractérisé en ce que** l'élément d'amenée est un corps creux ou un corps avec un rail de guidage (1b).

4. Dispositif selon l'une des revendications 1 à 3, **caractérisé en ce que** le système d'imagerie est un système MR, CT ou à ultrasons.

5. Dispositif selon l'une des revendications 1 à 4, **caractérisé en ce que** la table de patient (8) est déplaçable transversalement et/ou est apte à tourner.

6. Dispositif selon l'une des revendications 1 à 5, **caractérisé en ce que** le dispositif de support (9) permet le déplacement de la source de rayonnement (10) ou de l'élément d'amenée (1a) le long d'un rayon (11) relativement à la table de patient (8).

7. Dispositif selon l'une des revendications 1 à 6, **caractérisé en ce que** le dispositif de support (9) présente des sections (9a), axes (9b) ou articulations (9c) qui permettent un mouvement et une rotation transversale du rayonnement de la source de rayonnement (10) ou de l'élément d'amenée (1a).

8. Dispositif, en particulier selon l'une des revendications 1 à 7, **caractérisé en ce qu'**il est associé à celui-ci un dispositif d'amenée (12) pour le positionnement de l'instrument (4) dans le rayonnement électromagnétique orienté, pour le positionnement à l'élément d'amenée (1a) et/ou pour l'introduction de l'instrument le long de la trajectoire (6) entre la zone d'accès (2) et la zone visée (5).

9. Dispositif selon la revendication 7 ou 8, **caractérisé en ce que** le dispositif d'amenée (12) présente une griffe (13).
